(19) Europäisches Patentamt
European Patent Office
Office européen des brevets

(11) Numéro de publication: **0 263 039**
**A1**

(12)
# DEMANDE DE BREVET EUROPEEN

(21) Numéro de dépôt: **87420218.7**

(22) Date de dépôt: **10.08.87**

(51) Int. Cl.[4]: **A 61 K 6/10**
C 08 L 83/07
//(C08L83/07,83:05,83:07,83:07)

(30) Priorité: **12.08.86 FR 8611786**

(43) Date de publication de la demande:
**06.04.88 Bulletin 88/14**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Molteni, Carlo**
**Via Dei Bognetti, No. 9**
**I-20141 Milano (IT)**

(74) Mandataire: **Vogt, Bernard et al**
**RHONE-POULENC INTERSERVICES Service Brevets**
**Chimie Centre de Recherches de Saint-Fons B.P. 62**
**F-69192 Saint-Fons Cédex (FR)**

(54) **Composition pâteuse diorganopolysiloxane vulcanisable à température ambiante en un élastomère silicone utilisable en particulier en podologie et en art dentaire.**

(57) La présente invention concerne une composition pâteuse diorganopolysiloxane vulcanisable à température ambiante en un élastomère silicone, caractérisée en ce qu'elle comporte :

1. - 3 diorganopolysiloxanes vinylés dont une gomme et deux huiles de viscosité différente,
2. - un organohydrogénopolysiloxane,
3. - un catalyseur au platine,
4. - une charge choisie parmi l'alumine et l'hydroxyde d'aluminium,
5. - un plastifiant.

Les compositions selon l'invention sont utilisables notamment en podologie et dans l'art dentaire.

EP 0 263 039 A1

**Description**

COMPOSITION PATEUSE DIORGANOPOLYSILOXANE VULCANISABLE A TEMPERATURE AMBIANTE EN UN ELASTOMERE SILICONE UTILISABLE EN PARTICULIER EN PODOLOGIE ET EN ART DENTAIRE

La présente invention concerne une composition pâteuse diorganonopolysiloxane vulcanisable à température ambiante en un élastomère silicone et utilisable en particulier en podologie et en art dentaire.

Les compositions pâteuses selon l'invention font partie de la famille des compositions diorganopolysiloxane vulcanisable à température ambiante en présence d'un catalyseur métallique généralement à base de platine par des réactions de polyaddition entre des radicaux vinyle et des atomes d'hydrogène liés directement à des atomes de silicium des chaînes polysiloxanes. Ces compositions polyaddition sont connues depuis longtemps et sont décrites dans de nombreux brevets tels que les brevets américains US-A-3 220 972, US-A-3 284 406, US-A-3 436 366, US-A-3 697 473 et US-A-4 340 709.

A la connaissance de la demanderesse il n'est pas connu d'utiliser des compositons polyaddition en podologie. Par contre il est bien connu, notamment par les brevets britanniques GB-A-1 430 285, GB-A-1 492 616, GB-A-1 570 411, GB-A-2 091 748 et les brevets européens EP-A-152 887, EP-A-158 141, EP-A-162 211, EP-A-166 107 et EP-A-169 365 d'utiliser des compositions polyaddition en art dentaire pour réaliser des masses de surmoulage des empreintes ou des porte-empreintes de dents.

Une autre grande famille de composition pâteuse diorganopolysiloxane vulcanisable à température ambiante est la famille des compositions polycondensation comprenant généralement un diorganopolysiloxane à terminaisons silanol, un polyalcoxysilane ou un polyalcoxysiloxane et un catalyseur à l'étain.

Il est connu d'utiliser des compositions polycondensation coulantes et non pas pâteuses en podologie. Il est également connu depuis longtemps d'utiliser en art dentaire des compositions polycondensation (voir par exemple le brevet britannique GB-A-1 041 851).

La podologie comprend la thérapeutique des pieds et des orteils (doigts de pieds) au moyen d'actions mécaniques engendrées par des semelles ou des coussinets en contact avec au moins une partie de la surface des pieds et/ou des doigts de pieds en particulier entre les doigts de pieds. Ces semelles et coussinets constituent des appareillages podologiques dénommés orthèses utilisés en orthoplastie permettant notamment de corriger et de prévenir la déformation et les blessures par frottement des orteils et de la voûte plantaire. Il existe à l'heure actuelle en podologie un besoin d'une composition silicone vulcanisable à température ambiante qui présente l'ensemble des propriétés suivantes :

- une toxicité nulle ou quasi nulle,
- une conservation dans le temps (temps de stockage) suffisante,
- l'absence de dégagement gazeux ou de produits étrangers lors de la vulcanisation,
- une grande facilité d'emploi lors de la réalisation des semelles ou coussinets ci-dessus,
- un très faible retrait,
- une bonne résistance à l'abrasion,
- une dureté suffisante.

La présente invention a résolu le problème ci-dessus se présentant spécifiquement en podologie, en proposant une composition diorganopolysiloxane pâteuse vulcanisable à température ambiante en un élastomère, caractérisée en ce qu'elle comporte :

1. - un mélange de trois diorganopolysiloxane vinylé comportant chacun au moins deux radicaux vinyle par molécule ::

1.a. - au moins une gomme diorganovinylorganopolysiloxane de formule :
$R'R_2SiO(R_2SXiO)_n(RR''SiO)_m SiR_2R'$ (1)
dans laquelle les radicaux R identiques ou différents sont choisis parmi un radical alkyle en $C_1$-$C_8$ inclusivement, éventuellement substitué par des atomes d'halogènes et des radicaux cyano, et un radical phényle, au moins 50 % en nombre des radicaux R étant des radicaux méthyle, R' et R'' est un radical vinyle ou un radical R avec la condition que si R' est un radical R, R'' est un radical vinyle et si R'' est R, R' est un radical vinyle, n et m sont des nombres entiers tels que la viscosité de la gomme soit supérieure à 1 000 000 mPa.s à 25°C, de préference comprise entre 5 000 000 et 30 000 000 mPa.s à 25 °C,

1.b. - au moins une huile diorganopolysiloxane de formule (1) ci-dessus dans laquelle R, R', R'' ont la signification donnée ci-dessus et n et m sont des nombres entiers choisis de telle sorte que la viscosité de l'huile soit comprise entre 20 000 et 200 000 mPa.s à 25 °C, de préférence comprise entre 50 000 et 150 000 mPa.s,

1.c. - au moins une huile diorganopolysiloxane de formule (1) ci-dessus dans laquelle R, R' et R'' ont la signification donnée ci-dessus et n et m sont des nombres entiers choisis de telle sorte que la viscosité de l'huile soit comprise entre 500 et 10 000 mPa.s à 25 °C, de préférence comprise entre 2 000 et 5 000 mPa.s.

2. - Au moins un organohydrogénopolysiloxane ayant une viscosité comprise entre 2 et 1 000 mPa.s à 25 °C, de préférence comprise entre 5 et 500 mPa.s, dont les radicaux organiques sont des radicaux R ayant la signification ci-dessus.

3. - Une quantité catalytiquement efficace d'un composé d'un métal du groupe du platine.

4. - Une charge choisie parmi l'hydroxyde d'aluminium séché et l'alumine.

5. - Une quantité efficace de plastifiant.

Les diorganopolysiloxane 1 sont des produits bien connus disponibles industriellement, de plus ils peuvent être fabriqués par de nombreux procédés décrits dans la littérature.

Les radicaux R des diorganopolysiloxane 1 peuvent être par exemple des radicaux méthyle, éthyle, propyle, isopropyle, butyle, pentyle, trifluoro-3,3,3 propyle, béta-cyanotéthyle et gamma-cyanopropyle.

A titre d'exemples concrets de motifs constituant les diorganopolysiloxane 1 on peut citer ceux de formules :

$(CH_3)_2$ SiO
$(CH_3)(C_6H_5)$ SiO
$CH_3(C_2H_5)SiO$
$CH_3CH_2CH_2(CH_3)$ SiO
$(CH_3)_3$ $SiO_{0,5}$
$(CH_3)_2CH_2 = CH$ $SiO_{0,5}$
$CH_3(C_6H_5)_2$ $SiO_{0,5}$
$CH_3(C_6H_5)(CH_2 = CH)$ $SiO_{0,5}$
$CH_3(CH_2 = CH)$ SiO
$C_6H_5(CH_2 = CH)$ SiO
$CH_3CH_2(CH_2 = CH)$ SiO

D'autres motifs, par exemple de formules $CH_3$ $SiO_{0,5}$ et $SiO_2$ peuvent être mélangés avec les motifs précédents, cependant ils représentent une faible quantité ne dépassant pas 2 % molaire de l'ensemble des motifs diorganosiloxyle et triorganosiloxyle.

Les diorganopolysiloxane 1 présentent donc des radicaux vinyle aux extrémités de chaîne, [motif terminal $(CH_2 = CH)R_2SiO_{0,5}$] et/ou dans la chaîne, [motif $(CH_2 = CH)RSiO$], les différents motifs diorganosiloxyle étant généralement répartis de façon statistique.

De préférence, la gomme 1.a. présente entre 0,005 et 0,5 mole % de ses motifs entrant dans sa composition choisis parmi ceux de formules $(CH_2 = CH)RSiO$ et $(CH_2 = CH)R_2SiO_{0,5}$.

Pour les huiles 1.b. et 1.c. cette teneur molaire en motif vinylé est respectivement comprise entre 0,05 et 3 moles % et entre 0,1 et 10 moles %.

L'organohydrogénopolysiloxane (2) peut être linéaire cyclique ou ramifié.

L'organohydrogénopolysiloxane (2) sensiblement linéaire ou ramifié possède des motifs de formule générale moyenne :

$$R_xH_y\,Si\,O_{\frac{4-x-y}{2}}$$

dans laquelle les radicaux R identiques ou différents ont la signification ci-dessus, mais la possibilité pour certains radicaux R de représenter des radicaux vinyle n'est pas exclue. De préférence au moins 80 % des radicaux R sont des radicaux méthyle.

Le symbole x représente un nombre quelconque allant de 1 à 1,99, le symbole y représente un nombre quelconque allant de 0,1 à 1, la somme x + y allant de 1,7 à 2,6. De préférence sont utilisés comme organohydrogénopolysiloxanes (2) des méthylhydrogénopolysiloxanes.

Les organohydrogénopolysiloxanes (2) sont disponibles sur le marché des silicones, de plus leurs techniques de préparation sont maintenant bien au point. L'une des techniques les plus utilisées consiste, dans un premier temps, à cohydrolyser des mélanges appropriés constitués de chlorosilanes choisis parmi ceux de formules : $R_3SiCl$, $R_2SiCl_2$, $RSiCl_3$, $SiCl_4$, $HR_2SiCl$, $HRSiCl_2$, $HSiCl_3$. Par mélanges appropriés doivent être compris des mélanges qui renferment chacun, par atome de silicium, un nombre de radicaux R et un nombre d'atomes d'hydrogène coïncidant respectivement avec les valeurs représentées par les symboles x et y de la formule générale moyenne, la somme de ces nombres devant également coïncider avec les valeurs permises pour la somme x + y.

Dans un deuxième temps les cohydrolysats sont portés à une température allant de 80 à 220 °C de préférence en présence d'agents acides tels que l'acide sulfurique, les terres activées par un acide. Lors de ce chauffage il se produit un réarrangement des liaisons siloxaniques ainsi que la condensation des groupes SiOH. Ces transformations conduisent aux polymères organohydrogénopolysiloxanes (2) qui possèdent ainsi, en fonction des mélanges de chlorosilanes de départ, des structures linéaires, cycliques ou ramifiées.

Parmi les polymères linéaires peuvent être cités, à titre illustratif, ceux répondant aux formules suivantes :

$(CH_3)_3Si[OSiH(CH_3)]_gOSi(CH_3)_3$,
$H(CH_3)_2Si[OSiH(CH_3)]_gOSi(CH_3)_2H$
$(CH_3)_3Si[OSiH(CH_3)]_g[OSi(CH_3)_2]_hOSi(CH_3)_3$
$H(CH_3)_2Si[OSiH(CH_3)]_g[OSi(CH_3)_2OSi(CH_3)_2H$
$(CH_3)_2C_2H_5[OSi(CH_3)H]_pOSi(CH_3)_3$
$(CH_3)_3Si[OSi(CH_3)H]_p[OSi(CH_3)C_2H_5]_qOSi(CH_3)_3$
$(CH_3)_2C_2H_5Si[OSi(CH_3)H]_pOSiC_2H_5(CH_3)_2$
$(CH_3)_2(n.C_3H_7)Si[OSi(CH_3)H]_pOSi(n.C_3H_7)(CH_3)_2$
$(CH_3)_3Si[OSi(C_2H_5)H]_{p'}[OSi(CH_3)_2]_{q'}OSi(CH_3)_3$
$(CH_3)_3Si[OSi(CH_3)H]_p[OSi(CH_3)CH=CH_2]_qO-Si(CH_3)_3$
$(CH_3)_2CH_2=CHSi[OSi(CH_3)H]_pO-SiCH=CH_2(CH_3)_2$

dans lesquelles le symbole g représente un nombre quelconque allant de 3 à 120, h un nombre quelconque allant de 1 à 50, p un nombre quelconque allant de 6 à 60, p' un nombre quelconque allant de 1 à 15, q un nombre quelconque allant de 1 à 10, q' un nombre quelconque allant de 7 à 40.

Ces polymères linéaires possèdent généralement une viscosité peu élevée, elle s'échelonne par exemple de 5 mPa.s à 500 mPa.s à 25 °C.

Parmi les polymères cycliques peuvent être cités à titre illustratif ceux répondant aux formules suivantes :

$[OSi(CH_3)H]_4$, $[OSi(CH_3)H]_5$, $[OSi(CH_3)H]_6$, $[OSi(CH_3)H]_3$,
$[OSi(CH_3)H]_3[OSi(CH=CH_2|CH_3]$, $[OSi(C_2H_5)H]_3$

Quant aux polymères ramifiés ils sont constitués chacun d'une combinaison de motifs choisis parmi ceux de formules $R_3SiO_{0,5}$, $R_2SiO$, $RSiO_{1,5}$, $SiO_2$, $HR_2SiO_{0,5}$, $HRSiO$, $HSiO_{1,5}$, chaque combinaison qui définit un polymère renferme au moins un motif choisi parmi ceux de formules $RSiO_{1,5}$, $SiO_2$, $HSiO_{1,5}$, les motifs étant cependant répartis d'une manière telle que la formule moyenne, ramenée à un silicium, de chaque polymère est englobée dans la formule générale moyenne précitée.

La viscosité de ces polymères s'étale de 2 mPa.s à 25 °C à 10 000 mPa.s à 25 °C.

A titre d'exemples concrets de polymères ramifiés de faible viscosité, peuvent être cités :

- ceux répondant aux formules ci-après :

$CH_3Si[OSi(CH_3)_2H]_3$, $Si[OSi(CH_3)_2H]_4$

$HSi[OSi(CH_3)_3][OSi(CH_3)_2H]_2$, $n.C_3H_7Si[O-Si(CH_3)_2H]_3$

$Si[OSi(CH_3)(C_2H_5)H][OSi(CH_3)_2H]_3$

- ceux constitués de motifs $SiO_2$ et $H(CH_3)_2SiO_{0,5}$de rapport $CH_3/Si$ 1 à 1,5.

Généralement les constituants (1) et (2) se trouvent dans la composition en quantité telle que le rapport en nombre des groupes SiH sur les groupes SiVi soit compris entre 0,5 et 5, de préférence entre 0,6 et 2,5.

On peut utiliser comme catalyseur (3) des composés d'un métal du groupe du platine, enparticulier leurs sels et complexes notamment les complexes de platine-oléfine comme décrit dans les brevets US-A-3 159 601 et US-A-3 159 662, les produits de réaction des dérivés du platine avec des alcools, des aldéhydes et des éthers décrits dans le brevet US-A-3 220 972, les catalyseurs platine-vinylsiloxane décrits dans les brevets français FR-A-1 313 846 et son addition 88 676 et le brevet français FR-A-1 480 409 ainsi que les complexes décrits dans les brevets américains US-A-3 715 334, US-A-3 775 452 et US-A-3 814 730 et les brevets français FR-A-2 575 085 et FR-A-2 575 086, ainsi qu'un catalyseur au rhodium tel que décrit dans les brevets US-A-3 296 291 et US-A-3 928 629.

Les métaux préférés du groupe du platine sont le platine et le rhodium ; le ruthénium bien que moins actif mais moins cher, est également utilisable.

On utilise généralement de 5 à 1 000 ppm, de préférence de 50 à 400 ppm, de catalyseur calculés en poids de métal par rapport au poids des polysiloxanes 1 et 2.

La charge 4 utilisée dans la composition de l'invention est de l'alumine finement broyée dont la taille de particule est comprise entre 0,1 et 50 µm, de préférence entre 1 et 20 µm. L'alumine utilisable peut être de l'alumine hydratée ou au moins partiellement déshydratée. On peut également utiliser l'hydroxyde d'aluminium séché, fabriqué par précipitation par voie humide, de granulométrie analogue à celle de l'alumine indiquée ci-dessus. L'intérêt principal de ces charges est de ne pas inhiber l'action catalytique du catalyseur.

Jusqu'à 50 % en poids, de préférence jusqu'à 20 % en poids des charges 4 peuvent être remplacées par des charges siliceuses diluantes de surface spécifique inférieure généralement à 50 $m^2/g$, de granulométrie analogue à celle de l'alumine indiquée ci-dessus comme par exemple le quartz broyé et les terres de diatomées.

Comme plastifiant 5 on peut utiliser des plastifiants liquides à température ambiante tels que de l'huile de vaseline, de l'huile de paraffine, du dioctylphtalate, des phosphates tels que le trioctylphosphate (FR-A-2 415 132), les polybutène (FR-A-2 256 231), des cycloalkylbenzène (FR-A-2 392 476), des alkylbenzène (FR-A-2 446 849) et un polymère diméthylpolysiloxane bloqué triméthylsiloxyle de viscosité comprise entre 2 et 80 mPa.s à 25 °C.

On peut également utiliser des plastifiants solides mais mous à température ambiante et présentant un point de fusion compris entre 30 et 60 °C. Des cires ou des microcires de paraffines conviennent plus particulièrement.

De façon préférée on utilise un plastifiant 5 constitué par un mélange de plastifiants liquides et solides définis ci-dessus, la quantité de plastifiant solide pouvant atteindre 30 % en poids du poids total de plastifiant 5 utilisé.

L'homme de métier n'aura aucune difficulté à choisir des quantités adaptées de constituants 1 à 5 en vue d'obtenir une composition pâteuse, non collante au toucher, vulcanisant en une durée comprise entre 3 et 10 mn et présentant une dureté Shore A comprise de préférence entre 30 et 60 en moins de 10 minutes.

De façon à assurer leur conservation et à présenter une tenue au stockage convenable, les compositions de l'invention sont conditionnées dans au moins deux composants ou emballages contenant les divers mélanges possibles des constituants 1 à 5, en évitant toutefois de conditionner dans le même emballage les constituants 2 et 3.

Des compositions convenant plus particulièrement comportent (en parties en poids de constituants 1 à 5) :

1. - 100 parties,
2. - 2 à 20 parties, de préférence 4 à 12,
3. - 5 à 1 000 ppm par rapport au poids de 1 et 2,
4. - 50 à 500 parties, de préférence 150 à 300,
5. - 5 à 100 parties, de préférence 10 à 50.

En ce qui concerne le constituant 1 on utilise de préférence pour 100 parties de 1.a., de 50 à 100 parties de 1.b. et de 20 à 60 parties de 1.c.

Les compositions selon l'invention conviennent tout particulièrement pour réaliser des semelles orthopédiques et des coussinets à placer sur ou entre les orteils pour corriger les ongles incarnés, pour prévenir l'apparition de durillons et d'oignons, pour corriger le chevauchement ou la déformation des orteils.

Les compositions selon l'invention sont également utilisables pour la prise d'empreintes du conduit auditif ou pour réaliser des bouchons du conduit auditif pour amortir les bruits ou pour protéger l'oreille interne de l'extérieur.

Un autre avantage des compositions selon l'invention réside en ce qu'elles sont également utilisables pour la production d'empreintes dentaires ou de porte-empreintes.

D'autres avantages et caractéristiques de la présente invention apparaîtront à la lecture de l'exemple suivant donné à titre illustratif, nullement limitatif.

- EXEMPLE 1 :

On prépare un premier empâtage (partie A) en mélangeant intimement les ingrédients suivants, les parties étant exprimées en poids :

1. - polysiloxane 1.a., 1.b., 1.c. :

1.a. - une gomme méthylvinyldiméthylpolysiloxane bloquée à chacune de ses extrémités par un motif diméthylvinylsiloxyle comportant dans la chaîne 360 ppm de radicaux vinyle sous la forme de motif méthylvinylsiloxyle et 120 ppm de radicaux vinyle en bout de chaîne sous la forme de motif diméthylvinylsiloxyle de viscosité de 10 000 000 mPa.s à 25 °C .......... 20 parties

1.b. - une huile diméthylpolysiloxane bloquée à chacune de ses extrémités par un motif diméthylvinylsiloxyle de viscosité 100 000 mPa.s à 25 °C contenant 0,08 % en poids de radicaux vinyle ..........17 parties

1.c. - une huile diméthylpolysiloxane bloquée à chacune de ses extrémités par un motif diméthylvinylsiloxyle de viscosité 3 500 mPa.s à 25 °C contenant 0,2 % en poids de radicaux vinyle ..........15 parties

Charges 4 :

4.a. - de l'alumine finement broyée de diamètre particulaire moyen compris entre 1 et 20 µm (alumine SH 100 commercialisée par RHONE-POULENC) .......... 130 parties

4.b. - de la terre de diatomées (cellite) de diamètre particulaire moyen compris entre 1 et 20 µm .......... 5 parties

5 Plastifiants :

5.a. - de l'huile de vaseline .......... 11 parties

5.b. - de la paraffine de point de fusion 55 °C .......... 3 parties

On ajoute à la partie A 200 ppm de platine métal calculés par rapport au poids total de polymère 1 des parties A et B sous la forme d'un complexe du platine préparé à partir de l'acide chloroplatinique et de divinyl-1,3 tétraméthyl-1,1,3,3 disiloxane comme décrit à l'exemple 6 du brevet américain US-A-3 814 730.

Partie B :

On mélange les ingrédients suivants (les parties étant en poids) en utilisant, sauf indications contraires, les mêmes ingrédients qu'à la partie A.

Polymère 1 :

1.a. - .......... 20 parties

1.b. - .......... 18 parties

1.c. - .......... 10 parties

Polymère 2 :

Copolymère hydrogénométhyldiméthylpolysiloxane comportant des motifs méthylhydrogénosiloxyle et diméthylsiloxyle et à terminaisons triméthylsiloxyle ayant environ 1 % en poids d'atomes d'hydrogène liés au silicone et une viscosité d'environ 45 mPa.s à 25 °C.

Charges 4 :

4.a. - .......... 70 parties

4.b. - .......... 40 parties

Plastifiants 5 :

5.a. - .......... 11 parties

5.b. - .......... 3 parties

On mélange les parties A et B en proportions équipondérales. Le temps utile d'utilisation de la composition pâteuse obtenue est de 5 minutes.

La réticulation complète à 25 °C a lieu au bout de 10 minutes avec une dureté Shore A 40.

Le retrait linéaire est de 0,02 % après six heures à 25 °C et de 0,09 % après 7 jours.

A l'aide de cette composition on réalise de petits coussinets pour la podologie que l'on place de façon adaptée à un patient ayant des ortiels qui se chevauchent. Au bout de deux mois d'utilisation par le patient, le coussinet a conservé sa forme initiale et n'a pas subi d'abrasion.

**Revendications**

1. - Composition diorganopolysiloxane pâteuse vulcanisable à température ambiante en un élastomère, caractérisée en ce qu'elle comporte :

1. - un mélange de trois diorganopolysiloxane vinylé comportant chacun au moins deux radicaux vinyle par molécule :

1.a. - au moins une gomme diorganopolysiloxane de formule :

$R'R_2SiO(R_2SXiO)_n rRR''SiO)_m SiR_2R'$ (1)

dans laquelle les radicaux R identiques ou différents sont choisis parmi un radical alkyle en $C_1$-$C_8$ inclusivement, éventuellement substitué par des atomes d'halogènes et des radicaux cyano et, un radical phényle, au moins 50 % en nombre des radicaux R étant des radicaux méthyle, R' et R" est un radical vinyle ou un radical R avec la condition que si R' est un radical R, R" est un radical vinyle et si R" est R, R' est un radical vinyle, n et m sont des nombres entiers tels que la viscosité de la gomme soit supérieure à 1 000 000 mPa.s à 25 °C, de préférence comprise entre 5 000 000 et 30 000 000 mPa.s à 25 °C.

1.b. - au moins une huile diorganopolysiloxane de formule (1) ci-dessus dans laquelle R, R', R" ont la signification donnée ci-dessus et n et m sont des nombres entiers choisis de telle sorte que la viscosité de l'huile soit comprise entre 20 000 et 200 000 mPa.s à 25 °C, de préférence comprise entre 750 000 et 150 000 mPa.s.

1.c. - au moins une huile diorganopolysiloxane de formule (1) ci-dessus dans laquelle R, R' et R" ont la signification donnée ci-dessus et n et m sont des nombres entiers choisis de telle sorte que la viscosité de l'huile soit comprise entre 500 et 10 000 mPa.s à 25 °C, de préférence comprise entre 2 00 et 5 000.

2. - Au moins un organohydrogénopolysiloxane ayant une viscosité comprise

entre 2 et 1 000 mPa.s à 25 °C, de préférence entre 5 et 500 mPa.s, dont les radicaux organiques sont des radicaux R ayant la signification ci-dessus.

3. - Une quantité catalytiquement efficace d'un composé d'un métal du groupe du platine.

4. - Une charge choisie parmi l'hydroxyde d'aluminium séché et l'alumine.

5. - Une quantité efficace de plastifiant.

2. - Composition selon la revendication 2, caractérisée en ce que jusqu'à 50 % en poids de la charge 4 est remplacé par des charges siliceuses diluantes.

3. - Composition selon les revendications 1 et 2, caractérisée en ce que les plastifiants 5 sont un mélange de plastifiant liquide et solide.

4.- Composition selon la revendication 3, caractérisée en ce que le plastifiant liquide est choisi parmi l'huile de vaseline, l'huile de paraffine, les cycloalkylbenzène, les alkylbenzène et un polymère diméthylpolysiloxane bloqué triméthylsiloxyle, de viscosité comprise entre 2 et 80 mPa.s à 25 °C.

5. - Composition selon l'une quelconque des revendications précédentes, caractérisée en ce qu'elle comporte en parties en poids de constituants 1 à 5 :

1. - 100 parties,

2. - 2 à 20 parties, de préférence 4 à 12,

3. - 5 à 1 000 ppm par rapport au poids de 1 et 2,

4. - 50 à 500 parties, de préférence 150 à 300,

5. - 5 à 100 parties, de préférence 10 à 50.

6. - Composition selon la revendication 5, caractérisée en ce que pour 100 parties de 1.a. elle comporte 50 à 150 parties de 1.b. et de 20 à 120 parties de 1.c.

7. - Utilisation de compositions telles que définies à l'une quelconque des revendications 1 à 6 à la réalisation d'orthèses en podologie, d'empreintes dentaires ou d'empreintes du conduit auditif.

Office européen des brevets

# RAPPORT DE RECHERCHE EUROPEENNE

Numero de la demande

EP 87 42 0218

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | EP-A-0 169 365 (BAYER)<br>* Revendications 1,9,10 * | 1 | A 61 K 6/10<br>C 08 L 83/07 //<br>(C 08 L 83/07<br>C 08 L 83:05<br>C 08 L 83:07<br>C 08 L 83:07 ) |
| A,D | GB-A-2 091 748 (A. KETTENBACH)<br>* Revendications 1,8,9 * | 1 | |
| A | US-A-4 216 140 (C. SIMIZU)<br>* Revendication 1 * | 1 | |
| A | FR-A-2 553 999 (G-C DENTAL INDUSTRIAL CORP.)<br>* Revendications 1,3,4,12 * | 1 | |
| | | | **DOMAINES TECHNIQUES RECHERCHES (Int. Cl.4)** |
| | | | A 61 K<br>C 08 L |

Le présent rapport a été établi pour toutes les revendications

| Lieu de la recherche | Date d'achèvement de la recherche | Examinateur |
|---|---|---|
| LA HAYE | 10-12-1987 | DEPIJPER R.D.C. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)